# EUROPEAN PATENT APPLICATION

(11) **EP 3 929 591 A1**
(43) Date of publication of application: **29.12.2021**
(21) Application number: 20760214.5
(22) Date of filing: 15.01.2020
(51) Int. Cl.: G01N 35/00, G01N 21/00, G01N 33/53

(54) **MAGNETIC PARTICLE CHEMOLUMINESCENCE IMMUNOASSAY ANALYZER DARK BOX DETECTION SYSTEM**

(30) Priority: 20.02.2019 CN 201910126677
(71) Applicant: Chongqing Keysmile Biological Technology Co., Ltd., Chongqing 401121 (CN)
(72) Inventor: HU, Xiaolei, Chongqing 401121 (CN); XIONG, Zhiling, Chongqing 401121 (CN)
(74) Representative: Vigand, Philippe
(86) International application number: PCT/CN2020/072124
(87) International publication number: WO 2020/168864

(57) **Abstract**

A dark box detection system for a magnetic particle chemiluminescence immunoassay analyzer is disclosed. The dark box detection system includes a base plate (6); a detection dark box (1) arranged on the base plate (6), wherein the detection dark box (1) is provided with a dark room (10) inside, the dark room (10) having an opening in one side; an assay cup holder (2) which is provided with a cup placement slot (20) on which an assay cup is placed, and is slidable relative to the detection dark box (1) to open or close an inlet/outlet port (11), wherein the assay cup holder (2) has a detection position and a cup placement position within a sliding stroke; when the assay cup holder (2) is positioned at the detection position, the cup placement slot (20) is located inside the dark room (10), and the inlet/outlet port (11) is closed; when the assay cup holder (2) is positioned at the cup placement position, the cup placement slot (20) is located outside of the dark room (10), and the inlet/outlet port (11) is opened; a driving mechanism (3) configured to drive the assay cup holder (2) to slide; a detection mechanism (4) configured to detect a luminescence amount of a liquid inside the assay cup in the dark room (10); and a transferring device configured to place the assay cup into the cup placement slot (20) or remove the assay cup from the cup placement slot (20). Due to the adoption of an open-door detection dark box structure that is relatively independent from a grasper, the mutual interference between substrate filling and a negative-pressure suction nozzle of the grasper is effectively avoided, thereby improving the reliability of grasper motions and detection results.

## Description

### TECHNICAL FIELD

The present invention relates to the field of medical detection equipment, and more particularly to a dark box detection system for a magnetic particle chemiluminescence immunoassay analyzer.

### BACKGROUND

Chemiluminescence immunoassay is mainly of a measure of detection using the specific reaction of an antigen and an antibody. Because of its capabilities of amplifying and displaying detection signals by using isotopes, enzymes, chemiluminescent substances, etc., the antigen or antibody in an experiment is linked to a certain enzyme to form an enzyme-labeled antigen or antibody. This enzyme-labeled antigen or antibody retains both its immunological activity and enzyme activity, and is washed and then added with a luminescent substrate. In a laboratory, a solution to be detected is usually detected in an assay cup. After the luminescent substrate is added, the assay cup is fed to a detection dark box for detection. Therefore, the luminescence immunoassay of the solution to be detected is realized by detecting a luminescence amount of the substance in the assay cup in the detection dark box. The existing detection systems usually adopt a method of sealing a detection black box by using a grasper. For example, in the Patent Application No. CN201721086253.0 entitled "HIGH-EFFICIENCY DARK BOX DETECTION DEVICE FOR CHEMILUMINESCENCE IMMUNOASSAY", a grasper is used to suck a cup under a negative pressure and put it in a detection dark box, while achieving airtight shading by means of the grasper. However, in the presence of a substrate filling port, it is likely to cause reagent suction due to a negative pressure habit of the grasper, which adversely affects the reliability of the grasper; or a filling amount of the substrate is inaccurate due to the negative pressure, which adversely affects the accuracy of detection results.

### SUMMARY

In view of this, the present invention provides a dark box detection system for a magnetic particle chemiluminescence immunoassay analyzer, which helps to improve the reliability of a grasper, while ensuring the accuracy of substrate filling and the accuracy of detection results.

The technical schemes of the present invention are as follows.

A dark box detection system for a magnetic particle chemiluminescence immunoassay analyzer is characterized by including:
a base plate;
a detection dark box, which is arranged on the base plate and provided with a dark room inside, the dark room being provided with an inlet/outlet port in one side;
an assay cup holder, which is arranged on the base plate and provided with a cup placement slot configured to place an assay cup, and is slidable relative to the detection dark box to open or close the inlet/outlet port, wherein the assay cup holder is provided with a detection position and a cup placement position within its sliding stroke; when the assay cup holder is positioned at the detection position, the cup placement slot is located inside the dark room, and the inlet/outlet port is closed; when the assay cup holder is positioned at the cup placement position, the cup placement slot is located outside of the dark room, and the inlet/outlet port is opened;
a driving mechanism configured to drive the assay cup holder to slide;
a detection mechanism configured to detect a luminescence amount of a liquid inside the assay cup in the dark room; and
a transferring device configured to place the assay cup into the cup placement slot or remove the assay cup from the cup placement slot.

With the above scheme, the dark room adopts an open-door structure relatively independent of the grasper. Compared with the existing structure, the assay cup in the dark room in this scheme will not be affected by the negative pressure of the grasper, a negative-pressure suction nozzle of the grasper will not be affected by the liquid either, and the corresponding substrate filling will not be affected by the negative pressure, which ensures the accuracy of the substrate filling amount and facilitates improving the reliability of detection results and grasper actions.

Preferably, the driving mechanism includes a screw rod motor which is located on one side of the detection dark box, wherein an extension direction of a screw rod of the screw rod motor is consistent with a sliding direction of the assay cup holder, and the assay cup holder is in screw-thread fit with the screw rod. With the above scheme, the movement and position retention of the assay cup holder are realized by the screw rod motor, providing simple results and easy implementation.

Preferably, a cup holder guide rail which is parallel to the screw rod is fixedly arranged below the screw rod; a cup holder slider which is in sliding fit with the cup holder guide rail is arranged on the cup holder guide rail; and the assay cup holder is fixedly connected with the cup holder slider. With the above scheme, it is beneficial to ensure the stability of the assay cup holder during the movement, and meanwhile, ensure the accuracy of the cup placement slot at each position as well as the reliability of passing through the inlet/outlet port.

Preferably a motor reset sensor is arranged above one end of the screw rod close to the screw rod motor; and a trigger sheet adapted to the motor reset sensor is arranged on the assay cup holder. With the above scheme, the cooperation of the trigger sheet and the motor reset sensor helps to ensure the stability when the cup placement slot is in the detection position, and ensure the consistency in an initial position of the screw rod motor every time it runs, thereby avoiding large position errors that may lead to failure of cup placement or cup grasping.

Preferably, the assay cup holder also has a cup discarding position within its sliding stroke, and one side of the cup placement slot is open to form a cup discarding opening; the transferring device includes a push arm movably arranged on the base plate, wherein the push arm is movable along an orientation of the cup discarding opening; when the assay cup holder is located at the cup discarding position, the push arm moves to push the assay cup in the cup placement slot out of the cup discarding opening. With the above scheme, the push arm is directly pushed out from the cup discarding opening through the push arm, which reduces the grasper motion procedures and improves the efficiency of cup discarding.

Preferably, the transferring device further includes a grasping mechanism, the grasping mechanism including a supporting beam and a suction nozzle; the supporting beam is provided with an X-direction driving assembly configured to drive the suction nozzle to move horizontally in a length direction of the supporting beam, and a Z-direction driving assembly configured to drive the suction nozzle to move in a height direction of the supporting beam; the supporting beam is located on a side directly opposite to the inlet/outlet port; and the supporting beam and the screw rod are perpendicular to each other. With the above scheme, the horizontal and vertical movements of the suction nozzle are realized by the X-direction and Z-direction driving assemblies, meeting the requirements of respective procedures in the detection process.

In order to reduce components of a power mechanism and a transmission mechanism, improve the overall compactness and reduce the economic cost, the X-direction driving assembly includes an X-direction sliding rail arranged in the length direction of the supporting beam, and an X-direction driving motor located at one end of the supporting beam; the X-direction sliding rail is provided with a first X-direction slider that is in sliding fit with the X-direction sliding rail; a vertical plate that is fixedly connected with the first X-direction slider is arranged on the first X-direction slider;

the Z-direction driving assembly includes a Z-direction sliding rail arranged in a height direction of the vertical plate; a Z-direction slider that is in sliding fit with the Z-direction sliding rail is arranged on the Z-direction sliding rail; a Z-direction driving motor configured to drive the Z-direction slider to slide along the Z-direction sliding rail is arranged on the vertical plate; and the suction nozzle is fixedly connected with the Z-direction slider.

Preferably, the push arm is in sliding fit with the X-direction sliding rail through a second X-direction slider; a metal member is arranged on the vertical plate, and an upper magnet that is directly opposite to the metal member is arranged on the push arm; when the first X-direction slider is driven by the X-direction driving motor to approach the second X-direction slider, the metal member and the upper magnet attract each other to drive the push arm to slide along the X-direction sliding rail. With this method of mutual attraction by magnetic forces, the linkage between the vertical plate and the push arm is realized. That is, the X-direction driving assembly can realize the movements of the push arm and complete the cup discarding operation.

Preferably, the X-direction sliding rail has a front limiting structure and a rear limiting structure that are arranged on the front and rear sides of the push arm respectively; and the push arm is provided with a lower magnet that is directly opposite to the front limiting structure. With the above scheme, the sliding stroke of the push arm is limited by means of the limiting structures on the front and rear sides; and meanwhile, the push arm may be separated from the vertical plate and located in a fixed state relative to the supporting beam by means of the rear limiting structure, so as to prevent the push arm from sliding or shaking together and causing interferences to other components.

Preferably, the detection dark box is provided with an outwardly extending baffle corresponding to the inlet/out port; the baffle is located on the side directly opposite to the cup discarding opening; when the assay cup holder is located at the cup placement position, the baffle is located directly in front of the cup discarding opening; and the assay cup holder is provided with a keep-away hole that is located in a position directly opposite to the baffle. With the above scheme, when the assay cup is put into the cup placement slot, the baffle can just block the cup discarding opening, such that the stability of the assay cup in the cup placement slot can be ensured, and the assay cup is effectively prevented from falling out of the cup discarding opening, thereby improving the reliability of the structure.

Compared with the prior art, the present invention has the following technical effects.

According to the dark box detection system for the magnetic particle chemiluminescence immunoassay analyzer which adopts the above technical schemes, an open-door detection dark box structure that is relatively independent of the grasper is mainly adopted, such that the mutual interference between substrate filling and the negative-pressure suction nozzle of the grasper is effectively avoided, thereby improving the reliability of the grasper motions and the detection results.

### BRIEF DESCRIPTION OF THE DRAWINGS

FIG. 1 is a schematic structural diagram of the present invention;
FIG. 2 is a schematic diagram of a mounting structure of an assay cup holder;
FIG. 3 is a schematic diagram of an internal structure of a detection dark box when a cup placement slot is located at a detection position;
FIG. 4 is a schematic structural diagram of the detection dark box;
FIG. 5 is a schematic structural diagram of the assay cup holder;
FIG. 6 is a schematic structural diagram in which the detection dark box cooperates with the assay cup holder;
FIG. 7 is a schematic structural diagram when the cup placement slot is located at the detection position;
FIG. 8 is a schematic structural diagram when the cup placement slot is located at a cup placement position;
FIG. 9 is a schematic structural diagram when the cup placement slot is located at a cup discarding position;
FIG. 10 is a schematic structural diagram of a grasping mechanism;
FIG. 11 is a schematic diagram of a mounting structure of a push arm;
FIG. 12 is a schematic structural diagram of the push arm; and
FIG. 13 is a side view of FIG. 12.

### DETAILED DESCRIPTION

The present invention will be further explained below in conjunction with the embodiments and the accompanying drawings.

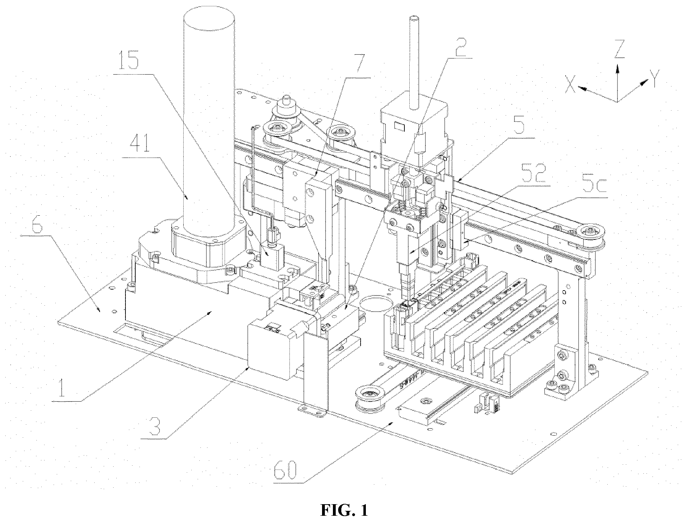

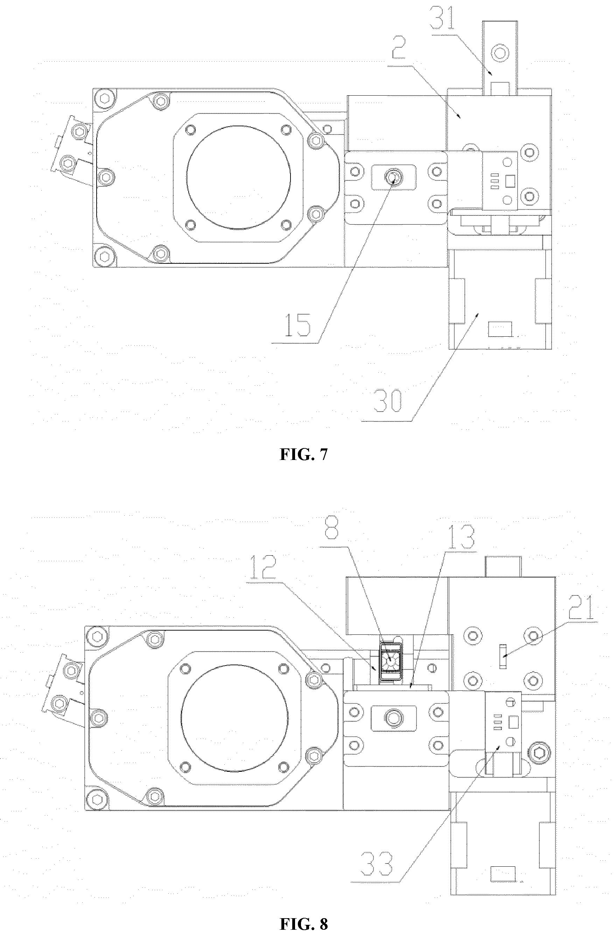

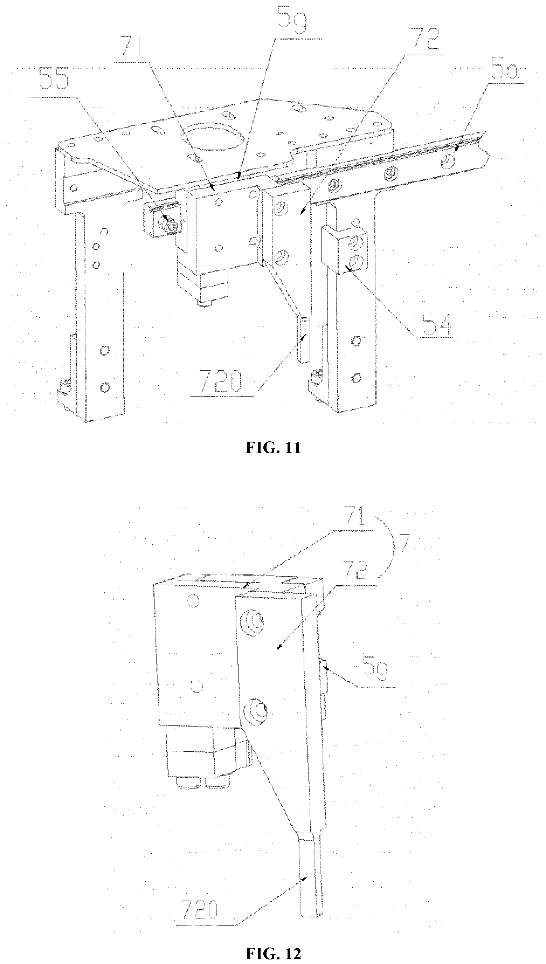

Referring to FIGs. 1 to 13, a dark box detection system for a magnetic particle chemiluminescence immunoassay analyzer of the present invention mainly includes a base plate 6 like a rectangular plate (for ease of understanding and literal introduction, in this embodiment, as shown in FIG. 1, a length direction of the base plate 6 is defined as an X direction, a width direction of the base plate 6 is defined as a Y direction, and a thickness direction of the base plate 6 is defined as a Z direction). An assay cup placement area 60 is arranged at one end of the base plate 6, and a detection dark box 1 is arranged at the other end of the base plate 6. The detection dark box 1 is provided with a hollow dark room 10 inside, wherein an inlet/outlet port 11 that is communicated with an inner cavity of the dark room 10 is formed in one side of the dark room 10. As shown in the figures, an orientation of the inlet/outlet port 11 is consistent with the Y direction. The base plate 6 is provided with an assay cup holder 2 at a position directly opposite to the inlet/outlet port 11, and is equipped with a driving mechanism 3 configured to drive the assay cup holder 2 to move. The assay cup holder 2 is slidable relative to the detection dark box 1 under the action of the driving mechanism 3, and has two positions for detection and cup placement on its sliding stroke. Meanwhile, the assay cup holder 2 is provided with a cup replacement slot 20 in which the assay cup is placed, and the top of the cup placement slot 20 is open.

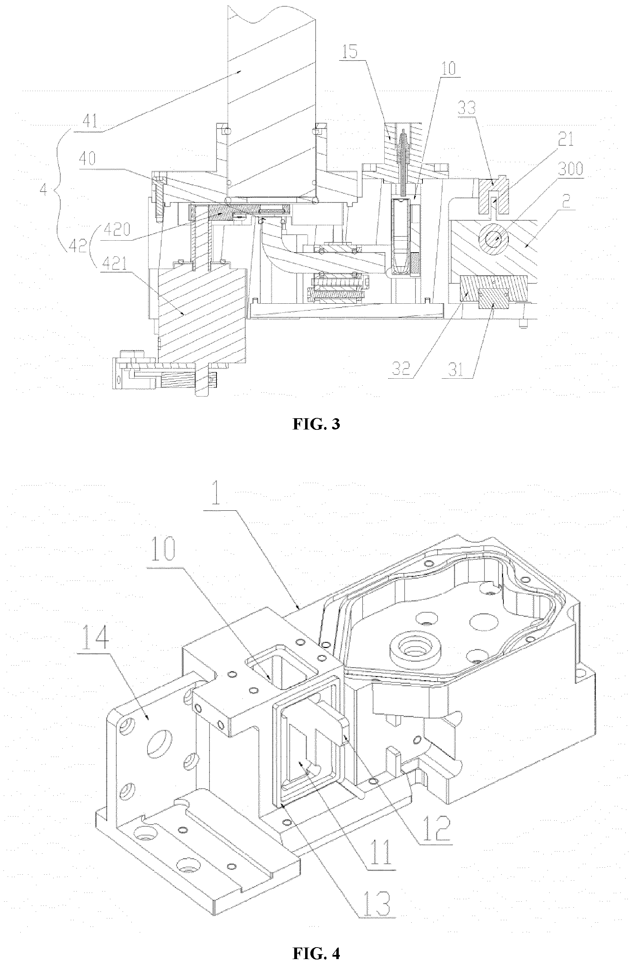

Referring to FIGs. 1 and 3, a substrate filling connector 15 is arranged at the top of the detection dark box 1, and communicated with the dark room 10. A substrate liquid may be filled through the substrate filling connector 15.

Referring to FIGs. 4 to 6, a sealing ring 13 having an annular structure is arranged at a position, corresponding to the inlet/outlet port 11, on the outer wall of the detection dark box 1. The sealing ring 13 protrudes vertically outward from the surface of the detection dark box 1, and is located on the circumferential outer side of the inlet/outlet port 11. A sealing groove 24 matched with the sealing ring 13 is arranged at a position, directly opposite to the sealing ring 13, on the assay cup holder 2. When the assay cup holder 2 is located at the detection position, the cup placement slot 20 is just located in the dark room 10, while the sealing ring 13 is embedded in the sealing groove 24, and the assay cup holder 2 is closely clung to a side wall directly opposite to the detection dark box 1, thereby realizing airtight shading of the dark room 10. When the assay cup holder 2 is located at the cup placement position, the cup placement slot 20 is located outside of the dark room 10, the corresponding sealing ring 13 is withdrawn from the sealing groove 24, and the inlet/outlet port 11 is in an open state.

Referring to FIGs. 1 to 5, a detection mechanism 4 is arranged on a side of the detection dark box 1 away from the assay cup placement area 60. The detection mechanism 4 is mainly configured to detect a luminescence amount of a solution in the assay cup in the dark room 10. The driving mechanism 3 is located on a side opposite to the detection mechanism 4, and an L-shaped mounting plate 14 is arranged on a side of the detection dark box 1 close to the assay cup placement area 60, and is integrally formed with the detection dark box 1.

The driving mechanism 3 includes a screw rod motor 30. As shown in the figures, the screw rod motor 30 is arranged in the Y direction and fixedly supported on the mounting plate 14, wherein an extension direction of a screw rod 300 of the screw rod motor 30 is consistent with an orientation of the inlet/outlet port 11. A cup holder guide rail 31 is arranged at a position, corresponding to the screw rod 300, at the bottom of the mounting plate 14. The assay cup guide rail 31 is arranged in the Y direction and provided with a cup holder slider 32 that is in sliding fit with the cup holder guide rail 31. The assay cup holder 2 is fixedly supported on the cup holder slider 32, and meanwhile, is in screw-thread fit with the screw rod 300 located above. When the screw rod motor 30 works, the assay cup holder 2 can be driven by the screw rod 300 to slide along the cup holder guide rail 31, enabling the assay cup holder 2 to stay at the detection position or the cup placement position.

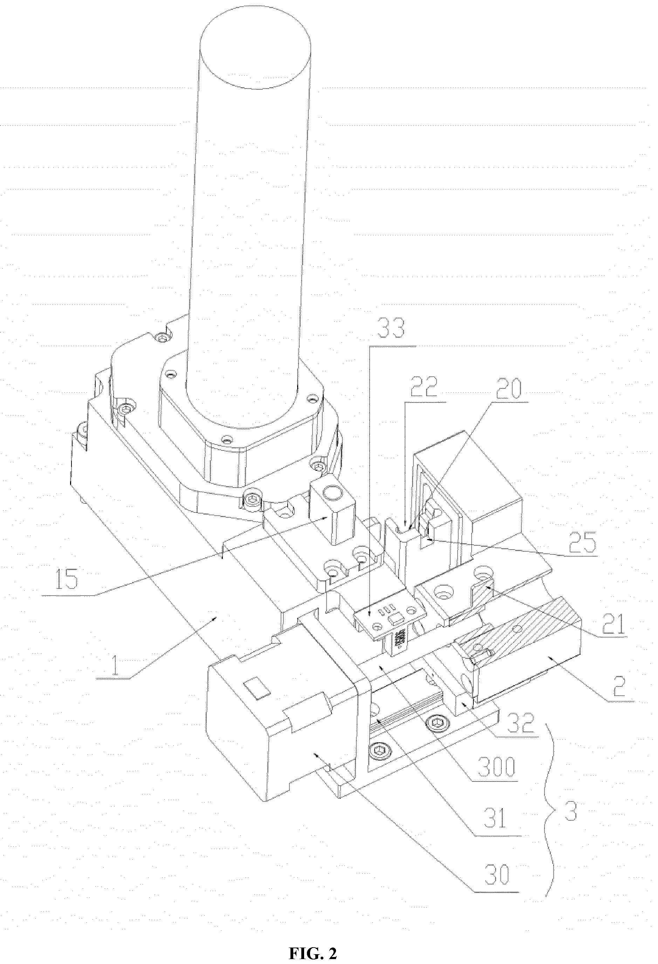

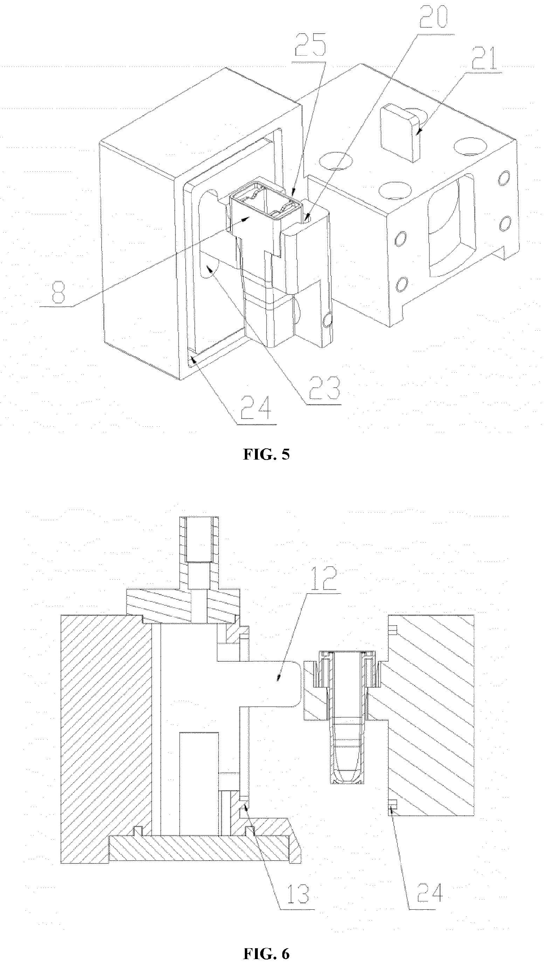

Referring to FIGs. 2 and 5, in this embodiment, in order to ensure the accurate staying position of the assay cup holder 2, a motor reset sensor 33 is arranged above the screw rod 300. As shown in the figures, the motor reset sensor 33 is fixed on the detection dark box 1, and is close to the root position of the screw rod 300. A trigger sheet 21 adapted to the motor reset sensor 33 is arranged on the assay cup holder 2. When the assay cup holder 2 is driven by the screw rod 300 to move toward the root of the screw rod 300 and the assay cup holder 2 is located at the detection position, the trigger sheet 21 just triggers the reset sensor 33. The reset sensor 33 may then send a reset signal to a central control of an instrument to continue the next step; otherwise, it is indicated that the assay cup holder 2 is not in place.

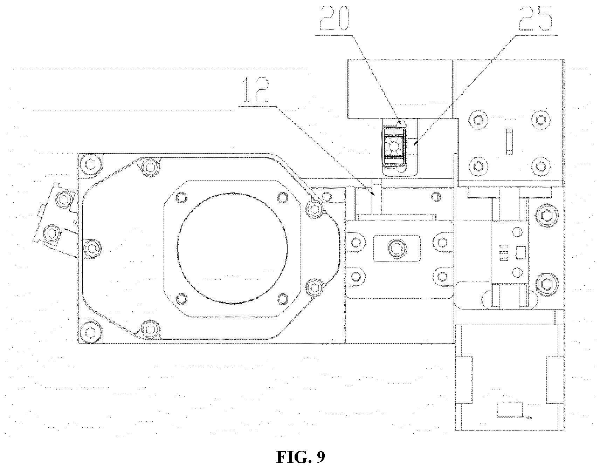

Referring to FIGs. 1, 2, 5, 9 and 10, in this embodiment, the assay cup holder 2 may be driven by the driving mechanism 3 to reach a cup discarding position shown in FIG. 9. The cup discarding position is farther away from the inlet/outlet port 11 than the cup placement position. Meanwhile, the cup placement slot 20 is open on the side away from the assay cup placement area 60 to form a cup discarding opening 22. The assay cup located in the cup placement slot 20 may be put in or taken out from the top opening, or may be moved in and out horizontally from the cup discarding opening 22. In this embodiment, the cup discarding opening 22 is mainly configured to remove the assay cup from the cup placement slot 20. Correspondingly, a transferring device configured to place the assay cup in the cup placement slot 20 and remove the assay cup from the cup placement slot 20 is arranged on the base plate 6.

As shown in the figures, the transferring device in this application further includes a grasping mechanism 5 and a push arm 7, wherein the grasping mechanism 5 includes a supporting beam 51 and a suction nozzle 52. The supporting beam 51 is arranged in the X direction which is perpendicular to an arrangement direction of the screw rod 300, and located on a side directly opposite to the inlet/outlet port 11. The supporting beam 51 is cooperatively provided with an X-direction driving assembly and a Z-direction driving assembly to drive the suction nozzle 52 to move in the X direction or the Z direction, thereby completing the actions of sucking the assay cup from the assay cup placement area 60, and then transporting the assay cup to the cup placement slot 20.

The X-direction driving assembly specifically includes an X-direction sliding rail 5a arranged in the length direction of the supporting beam 51. A first X-direction slider 5c which is in sliding fit with the X-direction sliding rail 5a is arranged on the X-direction sliding rail 5a. A vertical plate 53 fixedly connected with the first X-direction slider 5c is arranged on the first X-direction slider 5c. The vertical plate 53 is of a rectangular plate structure and is arranged in the Z direction. An X-direction driving motor 5b is arranged at one end of the supporting beam 51. In this embodiment, the X-direction driving motor 5b drives the first X-direction slider 5c to slide along the X-direction sliding rail 5a through a synchronous belt transmission mechanism, wherein a synchronous belt in the synchronous belt transmission mechanism is fixedly connected to the vertical plate 53.

The Z-direction driving assembly specifically includes a Z-direction sliding rail 5d arranged in a height direction of the vertical plate 53. The Z-direction sliding rail 5d is fixed on the vertical plate 53, and meanwhile, is provided with a Z-direction slider 5e in sliding fit with the Z-direction sliding rail 5d. A Z-direction driving motor 5f is arranged at the upper end of the vertical plate 53. In this embodiment, the Z-direction driving motor 5f is also a screw nut motor, which drives the screw rod to face vertically downward and to be in screw-thread fit with the Z-direction slider 5e; and the suction nozzle 52 is fixedly connected with the Z-direction slider 5e. In this way, when the Z-direction driving motor 5f works, the suction nozzle 52 can be driven to move vertically in the Z-direction.

In this embodiment, the push arm 7 is movably arranged on the X-direction sliding rail 5a through a second X-direction slider 5g. As shown in the figures, the push arm 7 is slidable in the X-direction along the X-direction sliding rail 5a through the second X-direction slider 5g. The push arm 7 mainly includes a push arm holder 71 and a push arm rod 72. The push arm holder 71 is fixedly connected to the second X-direction slider 5g, and the push arm rod 72 is then fixedly connected to the push arm holder 71. In addition, the push arm holder 71, the push arm rod 72 and the suction nozzle 52 mutually have a certain position offset in the Y direction, wherein a vertical distance between the push arm 72 and the suction nozzle 52 in the Y direction is adapted to a distance between the cup placement position and the cup discarding position.

The push arm rod 72 extends vertically downward, and has a toggle head 720 at its lower end. A U-shaped through hole 25 is formed in the upper end of a side wall, that faces the cup discarding opening 22, of the cup placement slot 20. The through hole 25 is adapted to the toggle head 720 in size, and is located on a sliding path of the toggle head 720. In this way, when the assay cup holder 2 is located at the cup discarding position, the push arm 7 slides in the X direction, and the toggle head 720 can enter the cup placement slot 20 via the through hole 25, thereby pushing the assay cup out of the cup discarding opening 22. A recycling hole is formed in a corresponding position of the base plate 6, and the pushed-out assay cup falls from the recycling hole and is recycled.

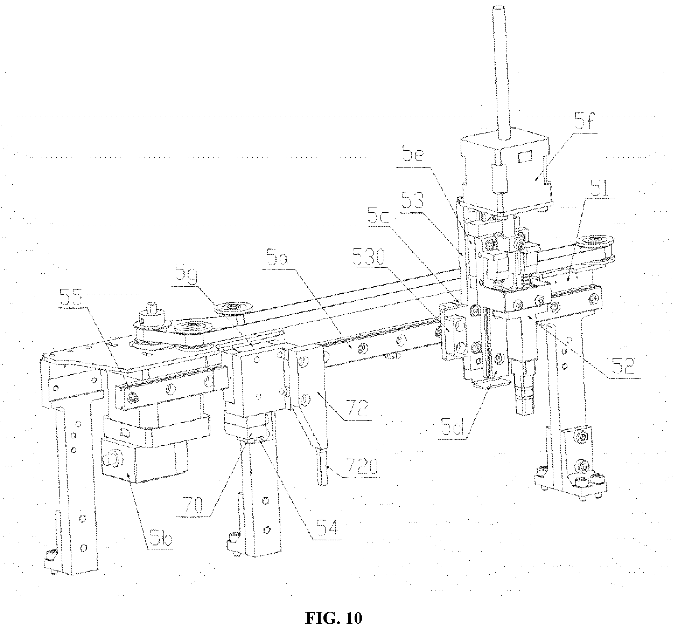

In order to simplify the structure and reduce the manufacturing cost, the sliding of the push arm 7 in this application is completed by the X-direction driving assembly of the grasper. As shown in FIGs. 10 to 13, magnets, i.e., an upper magnet 73 and a lower magnet 70 respectively, are arranged at the upper part and the lower part of one side of the push arm holder 71 close to the suction nozzle 52, respectively. A metal member 530 is arranged at a position, that is directly opposite to the upper magnet 73, on the vertical plate 53. When the vertical plate 53 slides together with the first X-direction slider 5c and approaches the push arm 7, the metal member 530 and the upper magnet 73 attract each other, which can drive the push arm 7 to slide in the X direction, thereby completing the actions of cup discarding and resetting.

On this basis, in order to prevent an interference of the push rod 72 to lower equipment caused by unnecessary sliding of the push arm 7, in this embodiment, a front limiting structure 54 and a rear limiting structure 55 are arranged on the X-direction sliding rail 5a. The front limiting structure 54 and the rear limiting structure 55 correspond to a position of the cup placement slot 20 in the X direction, and are slightly wider than the cup placement slot 20. A spacing between the front limiting structure 54 and the rear limiting structure 55 forms a sliding range of the push arm 7. As shown in the figures, in this embodiment, the rear limiting structure 55 is a limiting screw, which is located at the end of the X-direction sliding rail 5a and protrudes outward. The front limiting structure 54 is a limiting block arranged on the supporting beam 51; and the limiting block does not hinder the sliding of the vertical plate 53 in the X direction. In addition, the limiting block is also made of a metal flux magnetic material, and is arranged directly opposite to the lower magnet 70. When the suction nozzle 52 moves from the assay cup placement area 60 to the end where the detection dark box 1 is located, and after the vertical plate 53 approaches the push arm 7, the metal member 530 and the upper magnet 73 attract each other, which can drive the push arm 7 to slide together. However, if the first X-direction slider 5c slides reversely, that is, the first X-direction slider 5c slides from the end where the detection dark box 1 is located toward the assay cup placement area 60, when the first X-direction slider 5c slides to the front limiting structure 54, the lower magnet 70 and the limiting block abut against and attract each other, and the push arm 7 cannot continue to slide. Meanwhile, the upper magnet 73 and the metal member 530 are separated from each other, and the suction nozzle 52 continues to move. In addition, because the lower magnet 70 and the limiting block are attracted to each other, the push arm 7 will not sway relative to the supporting beam 51, which ensures that the push arm 7 stays at a reset position, without any interference on other components below.

Referring to FIGs. 4 to 9, a baffle 12 is arranged at a position, corresponding to the inlet/outlet port 11, on the detection dark box 1. As shown in the figures, the baffle 12 is located on the side directly opposite to the cup discarding opening 22, and is located at the upper part of the inlet/outlet port 11. The baffle 12 extends outward in the Y direction, and an extension length of the baffle 12 is adapted to the cup placement position of the assay cup holder 2. That is, when the assay cup holder 2 is located at the cup placement position, the baffle 12 is just located in front of the cup discarding opening 22, and is closely clung to the side wall of the cup placement slot 20, such that the assay cup placed in the cup placement slot 20 is prevented from falling out of the cup discarding opening 22 during the movement, thereby avoiding adverse influence on the detection efficiency. Correspondingly, in order to prevent the baffle 12 from interfering with the sliding of the assay cup holder 2, the assay cup holder 2 is provided with a keep-away hole 23 at a position directly opposite to the baffle 12. When the assay cup holder 2 moves from the cup placement position to the detection position, the baffle 12 can just be inserted into the keep-away hole 23. As the baffle 12 is located inside the sealing ring 13, the sealing performance of the inlet/outlet port 11 is not affected.

Referring to FIGs. 1 to 3, the detection mechanism 4 in this application mainly includes a light guide rod 40, a photoelectric tube 41, and a light control assembly 42. The light guide rod 40 is located in the detection dark box 1, wherein one end of the light guide rod is directly opposite to and communicated with the dark room 10. In addition, when the assay cup holder 2 is located at the detection position, this end is directly opposite to the lower part of the discarding cup opening 22, and is configured to guide the luminosity of the liquid in the assay cup in the cup placement slot 20 to the photoelectric tube 41. The other end of the light guide rod is directly opposite to a photosensitive end of the photoelectric tube 41. The light control assembly 42 mainly includes a light control plate 420 arranged between the light guide rod 40 and the photoelectric tube 41, and a light control driving motor 421 that drives the light control plate 420 to rotate horizontally. The light control driving motor 421 drives the light control plate 420 to rotate to realize on and off of a light path between the light guide rod 40 and the photoelectric tube 41, thereby achieving a better light control effect, avoiding damage to the photoelectric tube 41, and prolonging the service life of the photoelectric tube 41.

Referring to FIGs. 1 to 3, a working process of the present invention is as follows: the assay cup placement area 60 is mainly used for placing the assay cup 8. At the beginning, the assay cup holder 2 is located at the cup placement position, the suction nozzle 52 is lowered to suck the assay cup 8 from the assay cup placement area 60 under a negative pressure, and then slides in the X direction to a position over the cup placement slot 20; and then, the suction nozzle 52 is lowered, and the assay cup 8 is put into the cup placement slot 20.

Then, the screw motor 30 is reset, such that the assay cup holder 2 moves from the cup placement position to the detection position. Meanwhile, the assay cup 8 is brought into the dark room 10, while the inlet/outlet port 11 is closed. Subsequently, a substrate solution is added into the assay cup 8 through the substrate filling connector 15, and then the detection of liquid luminescence is completed by the detection mechanism 4.

At the end of detection, the screw motor 30 works to move the assay cup holder 2 from the detection position to the cup discarding position; and meanwhile, the X-direction driving motor 5b works, such that the vertical plate 53 approaches the push arm 7 and drives the push arm 7 to slide in a direction away from the assay cup placement area 60. The toggle head 720 extends into the cup placement slot 20 to push the assay cup 8 out of the cup discarding opening 22, so as to discharge the assay cup 8. Next, the assay cup holder 2 is driven by the screw rod motor 30 to return to the cup placement position, and meanwhile, the suction nozzle 52 returns to a position above the assay cup placement area 60 to prepare a next assay cup 8 for detection.

It should be eventually noted that the above descriptions are only preferred embodiments of the present invention. Under the hint of the present invention, a person of ordinary skill in the art can make a variety of similar expressions without violating the purpose and claims of the present invention, and such changes fall within the protection scope of the present invention.

## Claims

1. A dark box detection system for a magnetic particle chemiluminescence immunoassay analyzer, comprising:
a base plate (6);
a detection dark box (1) which is arranged on the base plate (6) and provided with a dark room (10) inside, the dark room (10) being provided with an inlet/outlet port on one side;
an assay cup holder (2) which is arranged on the base plate (6) and provided with a cup placement slot (20) on which an assay cup is placed, and is slidable relative to the detection dark box (1) to open or close the inlet/outlet port (11), wherein the assay cup holder (2) has a detection position and a cup placement position within its sliding stroke; when the assay cup holder (2) is positioned at the detection position, the cup placement slot (20) is located inside the dark room (10), and the inlet/outlet port (11) is closed; when the assay cup holder (2) is positioned at the cup placement position, the cup placement slot (20) is located outside of the dark room (10), and the inlet/outlet port (11) is opened;
a driving mechanism (3) configured to drive the assay cup holder (2) to slide;
a detection mechanism (4) configured to detect a luminescence amount of a liquid inside the assay cup in the dark room (10); and
a transferring device configured to place the assay cup into the cup placement slot (20) or remove the assay cup from the cup placement slot (20).

2. The dark box detection system for the magnetic particle chemiluminescence immunoassay analyzer according to claim 1, wherein the driving mechanism (3) comprises a screw rod motor (30) which is located on one side of the detection dark box (1), an extension direction of a screw rod (300) of the screw rod motor (30) is consistent with a sliding direction of the assay cup holder (2), and the assay cup holder (2) is in screw-thread fit with the screw rod (300).

3. The dark box detection system for the magnetic particle chemiluminescence immunoassay analyzer according to claim 2, wherein a cup holder guide rail (31) which is parallel to the screw rod (300) is fixedly arranged below the screw rod (300); a cup holder slider (32) which is in sliding fit with the cup holder sliding rail (31) is arranged on the cup holder guide rail (31); and the assay cup holder (2) is fixedly connected with the cup holder slider (32).

4. The dark box detection system for the magnetic particle chemiluminescence immunoassay analyzer according to claim 2 or 3, wherein a motor reset sensor (33) is arranged above one end of the screw rod (300) close to the screw rod motor (30); and a trigger sheet (21) adapted to the motor reset sensor (33) is arranged on the assay cup holder (2).

5. The dark box detection system for the magnetic particle chemiluminescence immunoassay analyzer according to claim 2, wherein the assay cup holder (2) also has a cup discarding position within its sliding stroke, and one side of the cup placement slot (20) is open to form a cup discarding opening (22); the transferring device comprises a push arm (7) movably arranged on the base plate (6), and the push arm (7) is movable along an orientation of the cup discarding opening (22); when the assay cup holder (2) is located at the cup discarding position, the push arm (7) moves to push the assay cup in the cup placement slot (20) out of the cup discarding opening (22).

6. The dark box detection system for the magnetic particle chemiluminescence immunoassay analyzer according to claim 5, wherein the transferring device further comprises a grasping mechanism (5) comprising a supporting beam (51) and a suction nozzle (52); the supporting beam (51) is provided with an X-direction driving assembly configured to drive the suction nozzle (52) to move horizontally in a length direction of the supporting beam (51), and a Z-direction driving assembly configured to drive the suction nozzle (52) to move in a height direction of the supporting beam (51); the supporting beam (51) is located on a side directly opposite to the inlet/outlet port (11); and the supporting beam (51) and the screw rod (300) are perpendicular to each other.

7. The dark box detection system for the magnetic particle chemiluminescence immunoassay analyzer according to claim 6, wherein the X-direction driving assembly comprises an X-direction sliding rail (5a) arranged in the length direction of the supporting beam (51), and an X-direction driving motor (5b) located at one end of the supporting beam (51); the X-direction sliding rail (5a) is provided with a first X-direction slider (5c) that is in sliding fit with the X-direction sliding rail (5a); a vertical plate (53) fixedly connected with the first X-direction slider (5c) is arranged on the first X-direction slider (5c);
the Z-direction driving assembly comprises a Z-direction sliding rail (5d) arranged in a height direction of the vertical plate (53); a Z-direction slider (5e) that is in sliding fit with the Z-direction sliding rail (5d) is arranged on the Z-direction sliding rail (5d); a Z-direction driving motor (5f) configured to drive the Z-direction slider (5e) to slide along the Z-direction sliding rail (5d) is arranged on the vertical plate (53); and the suction nozzle (52) is fixedly connected with the Z-direction slider (5e).

8. The dark box detection system for the magnetic particle chemiluminescence immunoassay analyzer according to claim 7, wherein the push arm (7) is in sliding fit with the X-direction sliding rail (5a) through a second X-direction slider (5g); a metal member (530) is arranged on the vertical plate (53), and an upper magnet (73) that is directly opposite to the metal member (530) is arranged on the push arm (7); when the first X-direction slider (5c) is driven by the X-direction driving motor (5b) to approach the second X-direction slider (5g), the metal member (530) and the upper magnet (73) attract each other to drive the push arm (7) to slide along the X-direction sliding rail (5a).

9. The dark box detection system for the magnetic particle chemiluminescence immunoassay analyzer according to claim 8, wherein the X-direction sliding rail (5a) is provided with a front limiting structure (54) and a rear limiting structure (55) that are arranged on the front and rear sides of the push arm (7), respectively; and the push arm (7) is provided with a lower magnet (70) directly opposite to the front limiting structure (54).

10. The dark box detection system for the magnetic particle chemiluminescence immunoassay analyzer according to claim 5, wherein the detection dark box (1) is provided with an outwardly extending baffle (12) corresponding to the inlet/out port (11); the baffle (12) is located on a side directly opposite to the cup discarding open (22); when the assay cup holder (2) is located in the cup placement position, the baffle (12) is located directly in front of the cup discarding opening (22); and the assay cup holder (2) is provided with a keep-away hole (23) that is located in a position directly opposite to the baffle (12).
